# EUROPEAN PATENT SPECIFICATION

(11) **EP 4 393 921 B1**
(45) Date of publication and mention of the grant of the patent: **15.04.2026**
(21) Application number: 22879853.4
(22) Date of filing: 30.05.2022
(51) Int. Cl.: C07D 471/04, A61P 35/00, A61K 31/4375

(54) **NALIDINONE COMPOUND AS FGFR4 INHIBITOR AND USE THEREOF**
NALIDINONVERBINDUNG ALS FGFR4-INHIBITOR UND VERWENDUNG DAVON
COMPOSÉ DE NALIDINONE EN TANT QU'INHIBITEUR DE FGFR4 ET UTILISATION ASSOCIÉE

(30) Priority: 14.10.2021 CN 202111197990
(43) Date of publication of application: 03.07.2024
(73) Proprietor: Wenzhou Medical University, Wenzhou, Zhejiang 325035 (CN)
(72) Inventor: LI, Xiaokun, Wenzhou, Zhejiang 325035 (CN); LIU, Zhiguo, Wenzhou, Zhejiang 325035 (CN); ZHEN, Xiaohui, Wenzhou, Zhejiang 325035 (CN); LIN, Li, Wenzhou, Zhejiang 325035 (CN); QIAN, Jianchang, Wenzhou, Zhejiang 325035 (CN)
(74) Representative: karo IP
(86) International application number: PCT/CN2022/095973
(87) International publication number: WO 2023/060904

(56) References cited:
- WO-A1-2018/113584
- WO-A1-2018/113584
- CN-A- 104 540 809
- CN-A- 105 307 657
- CN-A- 108 264 511
- CN-A- 108 264 511
- CN-A- 109 384 790
- CN-A- 109 745 325
- CN-A- 109 745 325
- CN-A- 113 912 602
- US-B2- 10 968 220
- FANG BO ET AL: "Design, synthesis, and biological evaluation of 1,6-naphthyridine-2-one derivatives as novel FGFR4 inhibitors for the treatment of colorectal cancer", EUROPEAN JOURNAL OF MEDICINAL CHEMISTRY, vol. 259, 1 November 2023 (2023-11-01), AMSTERDAM, NL, pages 115703, XP093222370, ISSN: 0223-5234, DOI: 10.1016/j.ejmech.2023.115703

## Description

### FIELD OF TECHNOLOGY

The present invention belongs to the field of pharmaceutical chemistry, and specifically relates to a 2-oxo-1,2-dihydro-1,6-naphthyridin-7-yl compound with FGFR4 as a target, a preparation method therefor and use thereof.

### BACKGROUND

At present, a series of patents for studying FGFR inhibitors have been disclosed, while few patents about selective inhibition of FGFR4 are disclosed. Documents CN108264511A, CN109745325A as well as WO2018/113584A1 disclose 1,6-naphthyridine-2-one derivatives as FGFR4 inhibitors. Compared Compared with the FGFR inhibitors, selective inhibitors of FGFR4 have the advantage of low toxicity. However, current research on the FGFR4 inhibitors against tumors, such as liver cancer, is far from enough, and it is still necessary to research and develop new FGFR4 inhibitors.

### SUMMARY

One of purposes of the present invention is to provide a 2-oxo-1,2-dihydro-1,6-naphthyridin-7-yl compound with FGFR4 as a target, a preparation method therefor and use thereof so as to solve the above technical problems. The compound can selectively inhibit FGFR4.

In one embodiment, the 2-oxo-1,2-dihydro-1,6-naphthyridin-7-yl compound is one of the following compounds, stereoisomers thereof, tautomers thereof, or pharmaceutically acceptable salts thereof:

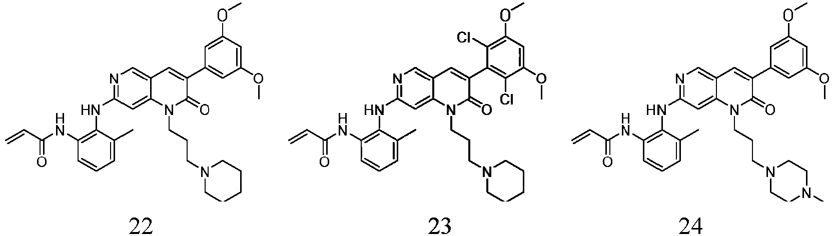

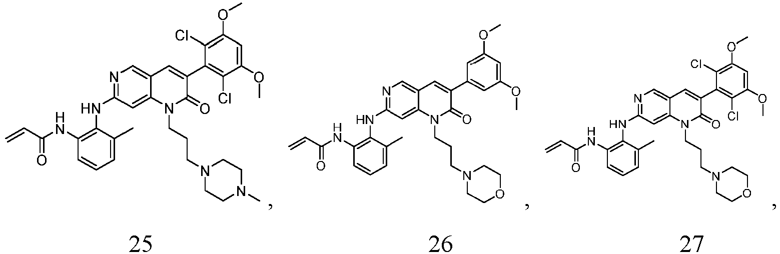

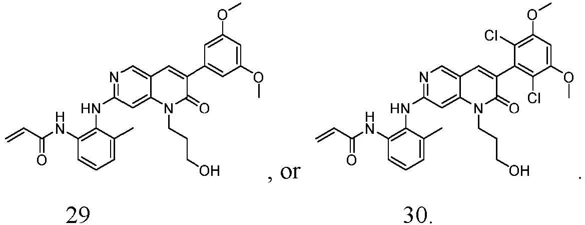

The present invention further provides the 2-oxo-1,2-dihydro-1,6-naphthyridin-7-yl compounds as disclosed above for use as drugs, characterized in that the drugs are antitumor drugs.

As a preference, the antitumor drugs use FGFR4 as a target. As a preference, the antitumor drugs selectively inhibit FGFR4, and have almost no inhibition on FGFR1, FGFR2 and FGFR3. As a preference, the drugs are used for treating liver cancer.

### BRIEF DESCRIPTION OF THE DRAWINGS

FIG. 1 shows proliferation inhibition activity of active compounds on hepatoma carcinoma cells HePG2 at a concentration of 20 µM;
FIG. 2 shows proliferation inhibition activity of active compounds on hepatoma carcinoma cells HuH-7 at a concentration of 20 µM;
FIG. 3 shows proliferation inhibition activity of active compounds on hepatoma carcinoma cells MIHA at a concentration of 20 µM;
FIG. 4 shows proliferation inhibition activity of an active compound 23 on colon cancer cells HCT-116 at a concentration of 3-100 µM;
FIG. 5 shows proliferation inhibition activity of the active compound 23 on colon cancer cells SW480 at a concentration of 3-100 µM; and
FIG. 6 shows proliferation inhibition activity of the active compound 23 on colon cancer cells RKO at a concentration of 3-100 µM.

### DESCRIPTION OF THE EMBODIMENTS

The present invention is further described below in combination with examples, but the examples are not intended to limit the scope of the present invention.

¹H NMR spectra are determined by a Bruker instrument (400 MHz), and chemical shifts are expressed in ppm. An expression method of ¹H NMR using tetramethylsilane as an internal standard (0.00 ppm) is as follows: s represents a single peak, d represents a double peak, t represents a triple peak, m represents a multiple peak, br represents a broadened peak, dd represents a double peak of a double peak, and dt represents a double peak of a triple peak. A coupling constant is provided in a unit of Hz. Mass spectra are determined by a liquid chromatography-mass spectrometry (LC/MS) instrument, and an ionization method may be electrospray ionization (ESI).

A Yantai Xincheng silica gel plate is used as a thin-layer chromatography silica gel plate, the silica gel plate used in thin-layer chromatography (TLC) has a specification of 0.2-0.25 mm, and a separation and purification product used in the thin-layer chromatography has a specification of 0.4-0.5 mm. In column chromatography, a 200- to 300-mesh Yantai silica gel is usually used as a carrier.

In the following examples, unless otherwise specified, all temperatures are centigrade temperatures; unless otherwise specified, various starting raw materials and reagents are commercially available or are synthesized according to known methods; and commercially available raw materials and reagents are directly used without further purification, unless otherwise specified.

CDCl₃ is deuterated chloroform; and DMSO-d6 is deuterated dimethyl sulfoxide. A hydrogen or nitrogen environment indicates that a reaction flask is connected with a balloon with a volume of about I L. In the examples, unless otherwise specified, a solution in a reaction refers to an aqueous solution.

Compounds are purified by silica gel column chromatography using an eluent system and thin-layer chromatography, where the eluent system is selected from: A: a petroleum ether and ethyl acetate system; and B: a dichloromethane and methanol system, volume ratios of solvents are different according to differences in the polarity of the compounds, and a small amount of an acidic or alkaline reagent, such as acetic acid or triethylamine, may also be added to conditions.

**Example 1** A route and a preparation process are as follows.

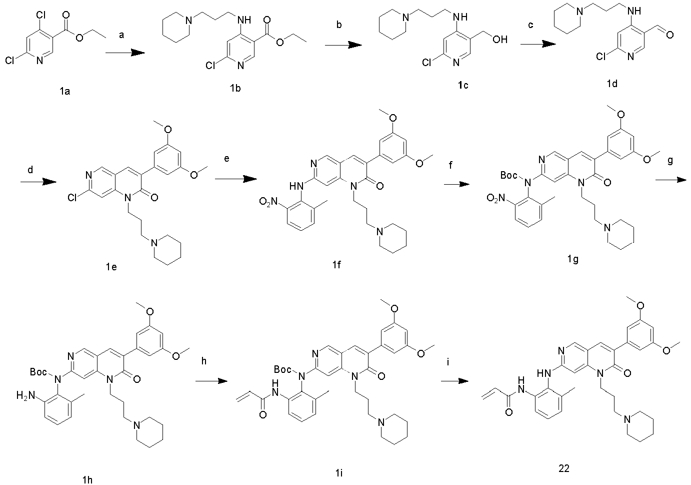

(1) Ethyl 4,6-dichloronicotinate (5 g, 22.72 mmol) and triethylamine (TEA) (9.45 ml, 68.17 mmol) were dissolved in tetrahydrofuran (THF), then 3-(4-piperidin-1-yl)propyl-1-amine (6.46 g, 45.44 mmol) was added dropwise to carry out a reaction overnight at room temperature, reduced pressure distillation was performed to remove the solvent, and a residue was purified by silica gel column chromatography (with an eluent: B system) to obtain ethyl 6-chloro-4-((3-(piperidin-1-yl)propyl)amino)nicotinate (5.6 g, a light yellow oily liquid) with a yield of 75.8%. (2) Lithium aluminum hydride (1.16 g, 30.69 mmol) was placed in a 500 ml two-necked flask, 100 ml of anhydrous THF was slowly added under the protection of nitrogen at -78°C, then the ethyl 6-chloro-4-((3-(piperidin-1-yl)propyl)amino)nicotinate (5 g, 15.35 mmol) (pre-dissolved in anhydrous THF) was slowly added dropwise to carry out a reaction at room temperature for 3 h, 2 ml of a saturated ammonium chloride solution was added dropwise at -78°C to quench the reaction (until a large amount of a solid was precipitated), then 3 ml of a 1 N NaOH solution was added and stirred at room temperature for 0.5 h, and suction filtration was performed to obtain a filtrate. Reduced pressure distillation was performed to remove the solvent, and a residue was purified by silica gel column chromatography (with an eluent: B system) to obtain (6-chloro-4-((3-(piperidin-1-yl)propyl)amino)pyridin-3-yl)methanol (3.1 g, a light yellow oily liquid) with a yield of 72.0%. (3) The (6-chloro-4-((3-(piperidin-1-yl)propyl)amino)pyridin-3-yl)methanol (3 g, 10.57 mmol) was put into anhydrous dichloromethane, manganese dioxide (9.19 g, 105.71 mmol) was added to carry out a reaction overnight at room temperature, suction filtration was performed to remove the manganese dioxide and obtain a filtrate, reduced pressure distillation was performed to remove the solution, and a residue was purified by silica gel column chromatography (with an eluent: B system) to obtain 6-chloro-4-((3-(piperidin-1-yl)propyl)amino)nicotinaldehyde (2.0 g, a light yellow oily liquid) with a yield of 68.6%. (4) The 6-chloro-4-((3-(piperidin-1-yl)propyl)amino)nicotinaldehyde (1.5 g, 5.32 mmol), K₂CO₃ (2.21 g, 15.97 mmol) and ethyl 2-(3,5-dimethoxyphenyl)acetate (1.79 g, 7.98 mmol) were put into 10 ml of anhydrous N,N-dimethylformamide (DMF) to carry out a reaction under the protection of nitrogen at 100°C for 12 h. The reaction system was dissolved in a large amount of ethyl acetate and then washed with a saturated sodium chloride solution, reduced pressure distillation was performed to remove the solution, and a residue was purified by silica gel column chromatography (with an eluent: B system) to obtain 7-chloro-3-(3,5-dimethoxyphenyl)-1-(3-(piperidin-1-yl)propyl)-1,6-naphthyridin-2(1H)-one (1.4 g, a light yellow oily compound) with a yield of 62.6%. (5) The 7-chloro-3-(3,5-dimethoxyphenyl)-1-(3-(piperidin-1-yl)propyl)-1,6-naphthyridin-2(1H)-one (1 g, 2.26 mmol), 2-nitro-6-methylaniline (516.41 mg, 3.39 mmol), Pd₂(dba)₃ (207.20 mg, 226.27 µmol), BrettPhos (242.91 mg, 452.53 µmol) and cesium carbonate (2.21 g, 6.79 mmol) were put into 30 ml of toluene to carry out reflux under the protection of nitrogen at 120°C for 6 h. Reduced pressure distillation was performed to remove the solvent toluene, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(3,5-dimethoxyphenyl)-7-((2-methyl-6-nitrophenyl)amino)-1-(3-(piperidin-1-yl)propyl)-1,6-naphthyridin-2(1H)-one (730 mg, a reddish brown solid) with a yield of 58.0%. (6) The 3-(3,5-dimethoxyphenyl)-7-((2-methyl-6-nitrophenyl)amino)-1-(3-(piperidin-1-yl)propyl)-1,6-naphthyridin-2(1H)-one (500 mg, 896.62 µmol), di-tert-butyl dicarbonate (391.37 mg, 1.79 mmol) and 4-dimethylaminopyridine (10.95 mg, 89.66 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(3,5-dimethoxyphenyl)-2-oxo-1-(3-(piperidin-1-yl)propyl)-1,2-2H-1,6-naphthyridin-7-yl)(2-methyl-6-nitrophenyl)carbamate (363 mg, a light yellow solid) with a yield of 61.50%. (7) The tert-butyl (3-(3,5-dimethoxyphenyl)-2-oxo-1-(3-(piperidin-1-yl)propyl)-1,2-2H-1,6-naphthyridin-7-yl)(2-methyl-6-nitrophenyl)carbamate (350 mg, 532.10 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-nitrophenyl)(3-(3,5-dimethoxyphenyl)-2-oxo-1-(3-(piperidin-1-yl)propyl)-1,2-2H-1,6-naphthyridin-7-yl)carbamate (136 mg, a white solid) with a yield of 40.8%. (8) The tert-butyl (2-amino-6-nitrophenyl)(3-(3,5-dimethoxyphenyl)-2-oxo-1-(3-(piperidin-1-yl)propyl)-1,2-2H-1,6-naphthyridin-7-yl)carbamate (100 mg, 159.29 µmol) and triethylamine (88.32 mmL, 637.16 µmol) were dissolved in anhydrous THF, and then acryloyl chloride (25.89 mmL, 318.58 µmol) were added dropwise to carry out a reaction at room temperature for 2 h. A saturated ammonium chloride solution was added to quench the reaction, reduced pressure distillation was performed to remove the solvent, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (2-acrylamide-6-nitrophenyl)(3-(3,5-dimethoxyphenyl)-2-oxo-1-(3-(piperidin-1-yl)propyl)-1,2-2H-1,6-naphthyridin-7-yl)carbamate (71 mg, a white solid) with a yield of 65.4%. (9) The tert-butyl (2-acrylamide-6-nitrophenyl)(3-(3,5-dimethoxyphenyl)-2-oxo-1-(3-(piperidin-1-yl)propyl)-1,2-2H-1,6-naphthyridin-7-yl)carbamate (50 mg, 73.33 µmol) was dissolved in 4 ml of dichloromethane, and 1 ml of trifluoroacetic acid was added to carry out a reaction at room temperature for 2 h. A saturated sodium bicarbonate solution was added to quench the reaction, then an ethyl acetate/saturated sodium chloride solution was used for extraction for several times, and organic layers were combined. Reduced pressure distillation was performed to remove the solvent, and a residue was purified by silica gel column chromatography (with an eluent: B system) to obtain a compound N-(2-((3-(3,5-dimethoxyphenyl)-2-oxo-1-(3-(piperidin-1-yl)propyl)-1,2-dihydro-1,6-naphthyridin-7-yl)amino)-3-methylphenyl)acrylamide (22 mg, a light yellow solid), recorded as a compound 22, with a yield of 52.7%. Characterization data are as follows: ¹H NMR (400 MHz, DMSO) δ 9.66 (s, 1H), 9.46 (s, 1H), 8.56 (s, 1H), 8.50 (s, 1H), 8.08 (s, 1H), 7.72 (d, J = 7.7 Hz, 1H), 7.25 (t, J = 7.8 Hz, 1H), 7.16 (d, J = 7.4 Hz, 1H), 6.87 (s, 2H), 6.51 (s, 1H), 6.23 (s, 1H), 5.69 (d, J = 10.4 Hz, 1H), 4.10 (s, 2H), 3.79 (s, 6H), 3.64 (s, 6H), 3.11 (s, 2H), 2.84 (dd, J = 21.1, 10.1 Hz, 2H), 2.21 (s, 3H), 1.99 (s, 2H), 1.81 (d, J = 13.6 Hz, 2H).

**Example 2** A route and a preparation process are as follows.

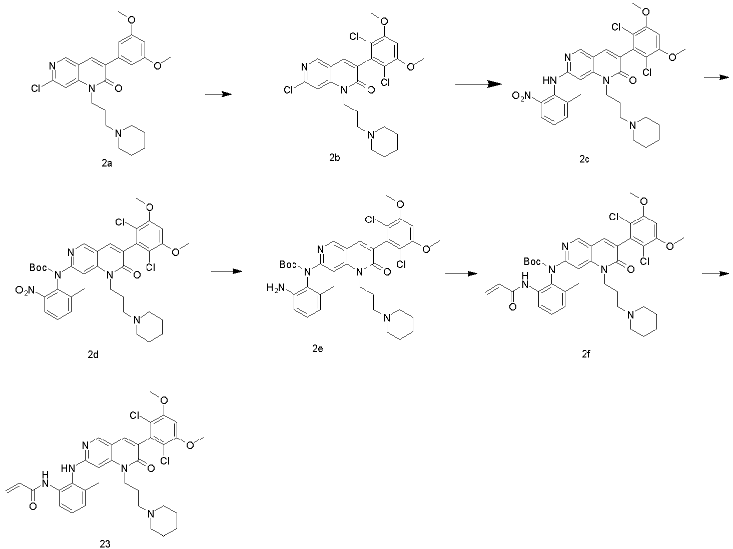

(1) 7-chloro-3-(3,5-dimethoxyphenyl)-1-(3-(piperidin-1-yl)propyl)-1,6-naphthyridin-2(1H)-one (1 g, 2.26 mmol) was dissolved in 50 ml of anhydrous acetonitrile and cooled to -20°C, and sulfonyl chloride (365.71 mmL, 4.53 mmol) (pre-dissolved in anhydrous acetonitrile) was added dropwise to carry out a reaction at room temperature for 10 min. A reaction solution was concentrated, 150 ml of dichloromethane was added, the pH value was adjusted to 8-9 with a saturated sodium bicarbonate solution, and layering was performed. An organic phase was concentrated, extraction was performed repeatedly with an ethyl acetate/distilled water system, and organic layers were combined. Reduced pressure distillation was performed to remove the solvent, and a residue was purified by silica gel column chromatography (with an eluent: B system) to obtain 7-chloro-3-(2,6-dichloro-3,5-dimethoxyphenyl)-1-(3-(piperidin-1-yl)propyl)-1,6-naphthyridin-2(1H)-one (877 mg, a white solid) with a yield of 75.6%. (2) The 7-chloro-3-(2,6-dichloro-3,5-dimethoxyphenyl)-1-(3-(piperidin-1-yl)propyl)-1,6-naphthyridin-2(1H)-one (877 mg, a white solid) (800 mg, 1.57 mmol), 2-nitro-6-methylaniline (357.42 mg, 2.35 mmol), Pd₂(dba)₃ (143.41 mg, 156.60 µmol), BrettPhos (168.12 mg, 313.21 µmol) and cesium carbonate (1.53 g, 4.70 mmol) were put into 30 ml of toluene to carry out reflux under the protection of nitrogen at 120°C for 6 h. Reduced pressure distillation was performed to remove the solvent, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(2,6-dichloro-3,5-dimethoxyphenyl)-7-((2-methyl-6-nitrophenyl)amino)-1-(3-(piperidin-1-yl)propyl)-1,6-naphthyridin-2(1H)-one (608 mg, a reddish brown solid) with a yield of 62.0%. (3) The 3-(2,6-dichloro-3,5-dimethoxyphenyl)-7-((2-methyl-6-nitrophenyl)amino)-1-(3-(piperidin-1-yl)propyl)-1,6-naphthyridin-2(1H)-one (500 mg, 798.04 µmol), di-tert-butyl dicarbonate (348.34 mg, 1.60 mmol) and 4-dimethylaminopyridine (9.75 mg, 79.80 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(2,6-dichloro-3,5-dimethoxyphenyl)-2-oxo-1-(3-(piperidin-1-yl)propyl)-1,2-2H-1,6-naphthyridin-7-yl)(2-methyl-6-nitrophenyl)carbamate (337 mg, a light yellow solid) with a yield of 58.2%. (4) The tert-butyl (3-(2,6-dichloro-3,5-dimethoxyphenyl)-2-oxo-1-(3-(piperidin-1-yl)propyl)-1,2-2H-1,6-naphthyridin-7-yl)(2-methyl-6-nitrophenyl)carbamate (300 mg, 412.85 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-methylphenyl)(3-(2,6-dichloro-3,5-dimethoxyphenyl)-2-oxo-1-(3-(piperidin-1-yl)propyl)-1,2-2H-1,6-naphthyridin-7-yl)carbamate (180 mg, a white solid) with a yield of 62.6%. (5) The tert-butyl (2-amino-6-methylphenyl)(3-(2,6-dichloro-3,5-dimethoxyphenyl)-2-oxo-1-(3-(piperidin-1-yl)propyl)-1,2-2H-1,6-naphthyridin-7-yl)carbamate (100 mg, 143.54 µmol) and triethylamine (79.59 mmL, 574.16 µmol) were dissolved in anhydrous THF, and then acryloyl chloride (23.33 mmL, 287.08 µmol) was added dropwise to carry out a reaction at room temperature for 2 h. A saturated ammonium chloride solution was added to quench the reaction, reduced pressure distillation was performed to remove the solvent, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (2-acrylamide-6-methylphenyl)(3-(2,6-dichloro-3,5-dimethoxyphenyl)-2-oxo-1-(3-(piperidin-1-yl)propyl)-1,2-2H-1,6-naphthyridin-7-yl)carbamate (73 mg, a white solid) with a yield of 68.2%. (6) The tert-butyl (2-acrylamide-6-methylphenyl)(3-(2,6-dichloro-3,5-dimethoxyphenyl)-2-oxo-1-(3-(piperidin-1-yl)propyl)-1,2-2H-1,6-naphthyridin-7-yl)carbamate (50.00 mg, 66.60 µmol) was dissolved in 4 ml of dichloromethane, and 1 ml of trifluoroacetic acid was added to carry out a reaction at room temperature for 2 h. A saturated sodium bicarbonate solution was added to quench the reaction, and then an ethyl acetate/saturated sodium chloride solution was used for extraction for several times. Reduced pressure distillation was performed to remove the solvent, and a residue was purified by silica gel column chromatography (with an eluent: B system) to obtain N-(2-((3-(2,6-dichloro-3,5-dimethoxyphenyl)-2-oxo-1-(3-(piperidin-1-yl)propyl)-1,2-2H-1,6-naphthyridin-7-yl)amino)-3-methylphenyl)acrylamide (26 mg, a light yellow solid), recorded as a compound 23, with a yield of 61.0%. Characterization data are as follows: ¹H NMR (400 MHz, DMSO) δ 9.61 (s, 1H), 9.21 (s, 1H), 8.55 (s, 1H), 8.48 (s, 1H), 7.79 (s, 1H), 7.71 (d, J = 7.7 Hz, 1H), 7.25 (t, J = 7.8 Hz, 1H), 7.17 (d, J = 7.4 Hz, 1H), 7.00 (s, 1H), 6.50 (dd, J = 17.0, 10.2 Hz, 1H), 6.23 (s, 1H), 5.69 (d, J = 10.3 Hz, 1H), 4.11 (s, 2H), 3.97 (s, 6H), 3.53 - 3.47 (m, 1H), 3.46 - 3.41 (m, 1H), 3.37 (d, J = 11.8 Hz, 2H), 3.01 (s, 2H), 2.83 (dd, J = 21.4, 10.1 Hz, 2H), 2.22 (s, 3H), 1.98 (s, 2H), 1.80 (d, J = 14.0 Hz, 2H), 1.72 - 1.62 (m, 2H).

**Example 3** A route and a preparation process are as follows.

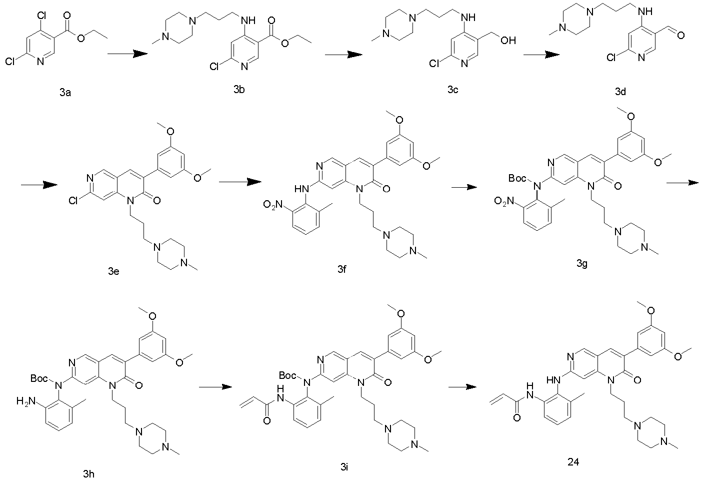

### (1) Ethyl 6-chloro-4-((3-(4-methylpiperazin-1-yl)propyl)amino)nicotinate

Ethyl 4,6-dichloronicotinate (5 g, 22.72 mmol) and triethylamine (TEA) (9.45 ml, 68.17 mmol) were dissolved in tetrahydrofuran (THF), then 3-(4-methylpiperazin-1-yl)propyl-1-amine (7.15 g, 45.44 mmol) was added dropwise to carry out a reaction overnight at room temperature, reduced pressure distillation was performed to remove the solvent, and a residue was purified by silica gel column chromatography (with an eluent: B system) to obtain ethyl 6-chloro-4-((3-(4-methylpiperazin-1-yl)propyl)amino)nicotinate (5.2 g, a light yellow oily liquid) with a yield of 68.0%. (2) Lithium aluminum hydride (1.11 g, 29.34 mmol) was placed in a 500 ml two-necked flask, 100 ml of anhydrous THF was slowly added under the protection of nitrogen at -78°C, then the ethyl 6-chloro-4-((3-(4-methylpiperazin-1-yl)propyl)amino)nicotinate (5 g, 14.67 mmol) (pre-dissolved in anhydrous THF) was slowly added dropwise to carry out a reaction at room temperature for 3 h, 2 ml of a saturated ammonium chloride solution was added dropwise at -78°C to quench the reaction (until a large amount of a solid was precipitated), then 3 ml of a 1 N NaOH solution was added and stirred at room temperature for 0.5 h, and suction filtration was performed to obtain a filtrate. Reduced pressure distillation was performed to remove the solvent, and a residue was purified by silica gel column chromatography (with an eluent: B system) to obtain (6-chloro-4-((3-(4-methylpiperazin-1-yl)propyl)amino)pyridin-3-yl)methanol (3.1 g, a light yellow oily liquid) with a yield of 71.9 %. (3) The (6-chloro-4-((3-(4-methylpiperazin-1-yl)propyl)amino)pyridin-3-yl)methanol (3 g, 10.04 mmol) was put into anhydrous dichloromethane, manganese dioxide (8.73 g, 100.40 mmol) was added to carry out a reaction overnight at room temperature, suction filtration was performed to remove the manganese dioxide and obtain a filtrate, reduced pressure distillation was performed to remove the solution, and a residue was purified by silica gel column chromatography (with an eluent: B system) to obtain 6-chloro-4-((3-(4-methylpiperazin-1-yl)propyl)amino)nicotinaldehyde (1.9 g, a light yellow oily liquid) with a yield of 65.0%. (4) The 6-chloro-4-((3-(4-methylpiperazin-1-yl)propyl)amino)nicotinaldehyde (1.5 g, 5.05 mmol), K₂CO₃ (2.10 g, 15.16 mmol) and ethyl 2-(3,5-dimethoxyphenyl)acetate (1.70 g, 7.58 mmol) were put into 10 ml of anhydrous N,N-dimethylformamide (DMF) to carry out a reaction under the protection of nitrogen at 100°C for 12 h. The reaction system was dissolved in a large amount of ethyl acetate and then washed with a saturated sodium chloride solution, reduced pressure distillation was performed to remove the solution, and a residue was purified by silica gel column chromatography (with an eluent: B system) to obtain 7-chloro-3-(3,5-dimethoxyphenyl)-1-(3-(4-methylpiperazin-1-yl)propyl)-1,6-naphthyridin-2(1H)-one (1.3 g, a light yellow oily liquid) with a yield of 58.0%. (5) The 7-chloro-3-(3,5-dimethoxyphenyl)-1-(3-(4-methylpiperazin-1-yl)propyl)-1,6-naphthyridin-2(1H)-one (1 g, 2.19 mmol), 2-nitro-6-methylaniline (499.44 mg, 3.28 mmol), Pd₂(dba)₃ (200.39 mg, 218.83 µmol), BrettPhos (234.93 mg, 437.66 µmol) and cesium carbonate (2.14 g, 6.56 mmol) were put into 30 ml of toluene to carry out reflux under the protection of nitrogen at 120°C for 6 h. Reduced pressure distillation was performed to remove the solvent toluene, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(3,5-dimethoxyphenyl)-7-((2-methyl-6-nitrophenyl)amino)-1-(3-(4-methylpiperazin-1-yl)propyl)-1,6-naphthyridin-2(1H)-one (673 mg, a reddish brown solid) with a yield of 52.6%. (6) The 3-(3,5-dimethoxyphenyl)-7-((2-methyl-6-nitrophenyl)amino)-1-(3-(4-methylpiperazin-1-yl)propyl)-1,6-naphthyridin-2(1H)-one (500 mg, 873.11 µmol), di-tert-butyl dicarbonate (381.11 mg, 1.75 mmol) and 4-dimethylaminopyridine (10.67 mg, 87.31 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(3,5-dimethoxyphenyl)-1-(3-(4-methylpiperazin-1-yl)propyl)-2-oxo-1,2-2H-1,6-naphthyridin-7-yl)(2-methyl-6-nitrophenyl)carbamate (397 mg, a light yellow solid) with a yield of 67.5%. (7) The tert-butyl (3-(3,5-dimethoxyphenyl)-1-(3-(4-methylpiperazin-1-yl)propyl)-2-oxo-1,2-2H-1,6-naphthyridin-7-yl)(2-methyl-6-nitrophenyl)carbamate (300.00 mg, 445.91 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-nitrophenyl)(3-(3,5-dimethoxyphenyl)-1-(3-(4-methylpiperazin-1-yl)propyl)-2-oxo-1,2-2H-1,6-naphthyridin-7-yl)carbamate (110 mg, a white solid) with a yield of 38.3%. (8) The tert-butyl (2-amino-6-nitrophenyl)(3-(3,5-dimethoxyphenyl)-1-(3-(4-methylpiperazin-1-yl)propyl)-2-oxo-1,2-2H-1,6-naphthyridin-7-yl)carbamate (100 mg, 155.57 µmol) and triethylamine (86.26 mmL, 622.28 µmol) were dissolved in anhydrous THF, and then acryloyl chloride (25.29 mmL, 311.14 µmol) were added dropwise to carry out a reaction at room temperature for 2 h. A saturated ammonium chloride solution was added to quench the reaction, reduced pressure distillation was performed to remove the solvent, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (2-acrylamide-6-nitrophenyl)(3-(3,5-dimethoxyphenyl)-1-(3-(4-methylpiperazin-1-yl)propyl)-2-oxo-1,2-2H-1,6-naphthyridin-7-yl)carbamate (67 mg, a white solid) with a yield of 61.7%. (9) The tert-butyl (2-acrylamide-6-nitrophenyl)(3-(3,5-dimethoxyphenyl)-1-(3-(4-methylpiperazin-1-yl)propyl)-2-oxo-1,2-2H-1,6-naphthyridin-7-yl)carbamate (50 mg, 71.75 µmol) was dissolved in 4 ml of dichloromethane, and 1 ml of trifluoroacetic acid was added to carry out a reaction at room temperature for 2 h. A saturated sodium bicarbonate solution was added to quench the reaction, then an ethyl acetate/saturated sodium chloride solution was used for extraction for several times, and organic layers were combined. Reduced pressure distillation was performed to remove the solvent, and a residue was purified by silica gel column chromatography (with an eluent: B system) to obtain a compound N-(2-((3-(3,5-dimethoxyphenyl)-2-oxo-1-(3-(4-methylpiperazin-1-yl)propyl)-1,2-dihydro-1,6-naphthyridin-7-yl)amino)-3-methylphenyl)acrylamide (21 mg, a light yellow solid), recorded as a compound 24, with a yield of 48.1%. Characterization data of the product are as follows: ¹H NMR (400 MHz, CDCl₃) δ 8.39 (s, 2H), 7.99 (d, J = 7.3 Hz, 1H), 7.69 (s, 1H), 7.28 (d, J = 6.6 Hz, 1H), 7.14 (d, J = 7.5 Hz, 1H), 6.77 (d, J = 2.2 Hz, 2H), 6.47 (t, J = 2.2 Hz, 1H), 6.31 (d, J = 16.9 Hz, 1H), 6.18 (dd, J = 16.9, 10.1 Hz, 1H), 5.88 (s, 1H), 5.67 (d, J = 9.0 Hz, 1H), 4.06 - 3.98 (m, 2H), 3.81 (s, 6H), 3.75 (s, 1H), 3.61 (s, 1H), 2.67 (s, 4H), 2.50 (s, 2H), 2.24 (s, 3H), 1.73 (s, 2H), 1.28 (s, 1H), 1.25 (s, 3H), 0.88 (t, J = 6.7 Hz, 1H).

**Example 4** A route and a preparation process are as follows.

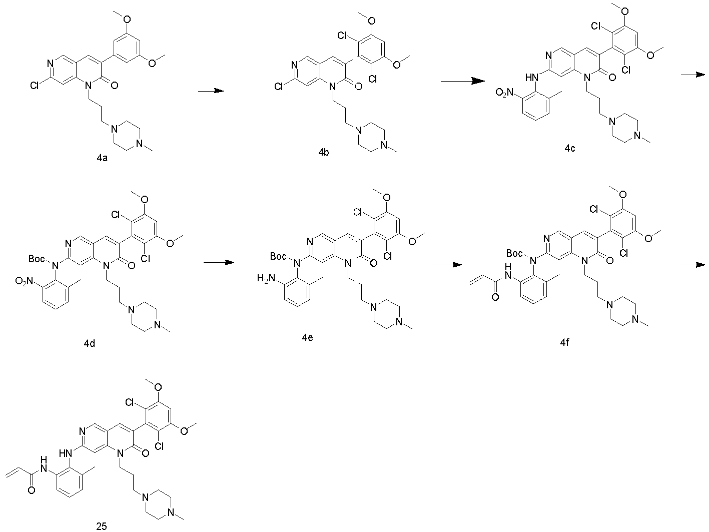

(1) 7-chloro-3-(3,5-dimethoxyphenyl)-1-(3-(4-methylpiperazin-1-yl)propyl)-1,6-naphthyridin-2(1H)-one (1 g, 2.19 mmol) was dissolved in 50 ml of anhydrous acetonitrile and cooled to -20°C, and sulfonyl chloride (353.69 mmL, 4.38 mmol) (pre-dissolved in anhydrous acetonitrile) was added dropwise to carry out a reaction at room temperature for 10 min. A reaction solution was concentrated, 150 ml of dichloromethane was added, the pH value was adjusted to 8-9 with a saturated sodium bicarbonate solution, and layering was performed. An organic phase was concentrated, extraction was performed repeatedly with an ethyl acetate/distilled water system, and organic layers were combined. Reduced pressure distillation was performed to remove the solvent, and a residue was purified by silica gel column chromatography (with an eluent: B system) to obtain 7-chloro-3-(2,6-dichloro-3,5-dimethoxyphenyl)-1-(3-(4-methylpiperazin-1-yl)propyl)-1,6-naphthyridin-2(1H)-one (785 mg, a white solid) with a yield of 78.5%. (2) The 7-chloro-3-(2,6-dichloro-3,5-dimethoxyphenyl)-1-(3-(4-methylpiperazin-1-yl)propyl)-1,6-naphthyridin-2(1H)-one (750.00 mg, 1.43 mmol), 2-nitro-6-methylaniline (325.51 mg, 2.14 mmol), Pd₂(dba)₃ (130.61 mg, 142.62 µmol), BrettPhos (153.12 mg, 285.25 µmol) and cesium carbonate (1.39 g, 4.28 mmol) were put into 30 ml of toluene to carry out reflux under the protection of nitrogen at 120°C for 6 h. Reduced pressure distillation was performed to remove the solvent, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(2,6-dichloro-3,5-dimethoxyphenyl)-7-((2-methyl-6-nitrophenyl)amino)-1-(3-(4-methylpiperazin-1-yl)propyl)-1,6-naphthyridin-2(1H)-one (534 mg, a reddish brown solid) with a yield of 58.4%. (3) The 3-(2,6-dichloro-3,5-dimethoxyphenyl)-7-((2-methyl-6-nitrophenyl)amino)-1-(3-(4-methylpiperazin-1-yl)propyl)-1,6-naphthyridin-2(1H)-one (500 mg, 779.36 µmol), di-tert-butyl dicarbonate (340.19 mg, 1.56 mmol) and 4-dimethylaminopyridine (9.52 mg, 77.94 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(2,6-dichloro-3,5-dimethoxyphenyl)-1-(3-(4-methylpiperazin-1-yl)propyl)-2-oxo-1,2-2H-1,6-naphthyridin-7-yl)(2-methyl-6-nitrophenyl)carbamate (362 mg, a light yellow solid) with a yield of 62.7%. (4) The tert-butyl (3-(2,6-dichloro-3,5-dimethoxyphenyl)-1-(3-(4-methylpiperazin-1-yl)propyl)-2-oxo-1,2-2H-1,6-naphthyridin-7-yl)(2-methyl-6-nitrophenyl)carbamate (300 mg, 404.49 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-methylphenyl)(3-(2,6-dichloro-3,5-dimethoxyphenyl)-1-(3-(4-methylpiperazin-1-yl)propyl)-2-oxo-1,2-2H-1,6-naphthyridin-7-yl)carbamate (165 mg, a white solid) with a yield of 57.6%. (5) The tert-butyl (2-amino-6-methylphenyl)(3-(2,6-dichloro-3,5-dimethoxyphenyl)-1-(3-(4-methylpiperazin-1-yl)propyl)-2-oxo-1,2-2H-1,6-naphthyridin-7-yl)carbamate (100 mg, 140.51 µmol) and triethylamine (77.91 mmL, 562.05 µmol) were dissolved in anhydrous THF, and then acryloyl chloride (22.84 mmL, 281.02 µmol) was added dropwise to carry out a reaction at room temperature for 2 h. A saturated ammonium chloride solution was added to quench the reaction, reduced pressure distillation was performed to remove the solvent, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (2-acrylamide-6-methylphenyl)(3-(2,6-dichloro-3,5-dimethoxyphenyl)-1-(3-(4-methylpiperazin-1-yl)propyl)-2-oxo-1,2-2H-1,6-naphthyridin-7-yl)carbamate (76 mg, a white solid) with a yield of 71.3%. (6) The tert-butyl (2-acrylamide-6-methylphenyl)(3-(2,6-dichloro-3,5-dimethoxyphenyl)-1-(3-(4-methylpiperazin-1-yl)propyl)-2-oxo-1,2-2H-1,6-naphthyridin-7-yl)carbamate (50.00 mg, 65.30 µmol) was dissolved in 4 ml of dichloromethane, and 1 ml of trifluoroacetic acid was added to carry out a reaction at room temperature for 2 h. A saturated sodium bicarbonate solution was added to quench the reaction, and then an ethyl acetate/saturated sodium chloride solution was used for extraction for several times. Reduced pressure distillation was performed to remove the solvent, and a residue was purified by silica gel column chromatography (with an eluent: B system) to obtain N-(2-((3-(2,6-dichloro-3,5-dimethoxyphenyl)-2-oxo-1-(3-(4-methylpiperazin-1-yl)propyl)-1,2-2H-1,6-naphthyridin-7-yl)amino)-3-methylphenyl)acrylamide (25 mg, a light yellow solid), recorded as a compound 25, with a yield of 58.3%. Characterization data are as follows: ¹H NMR (400 MHz, DMSO) δ 9.63 (s, 1H), 8.48 (s, 1H), 8.03 (s, 1H), 7.74 (s, 1H), 7.25 (s, 1H), 7.17 (s, 1H), 6.51 (t, J = 13.6 Hz, 2H), 5.69 (d, J = 10.1 Hz, 1H), 4.02 (s, 2H), 3.79 (s, 6H), 3.45 (s, 2H), 2.45 (s, 4H), 2.19 (s, 4H), 2.01 (s, 2H), 1.71 (s, 3H).

**Example 5** A route and a preparation process are as follows.

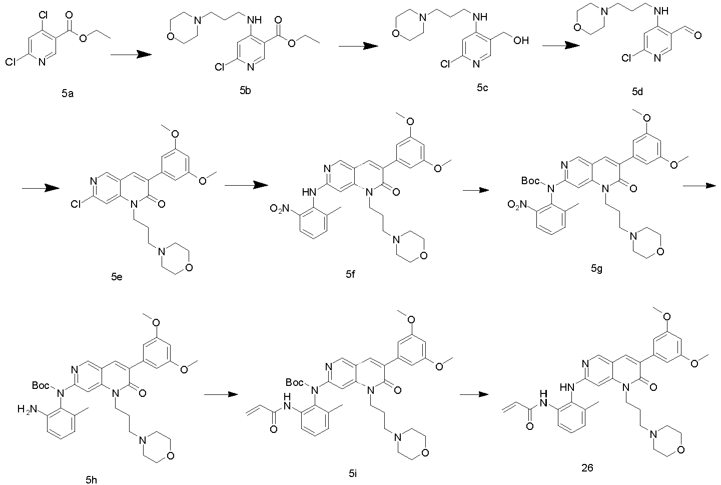

(1) Ethyl 4,6-dichloronicotinate (5 g, 22.72 mmol) and triethylamine (TEA) (9.45 ml, 68.17 mmol) were dissolved in tetrahydrofuran (THF), then 3-(4-morpholin-1-yl)propyl-1-amine (6.55 g, 45.44 mmol) was added dropwise to carry out a reaction overnight at room temperature, reduced pressure distillation was performed to remove the solvent, and a residue was purified by silica gel column chromatography (with an eluent: B system) to obtain ethyl 6-chloro-4-((3-morpholinopropyl)amino)nicotinate (5.8 g, a light yellow oily liquid) with a yield of 78.2%. (2) Lithium aluminum hydride (1.16 g, 30.51 mmol) was placed in a 500 ml two-necked flask, 100 ml of anhydrous THF was slowly added under the protection of nitrogen at -78°C, then the ethyl 6-chloro-4-((3-morpholinopropyl)amino)nicotinate (5 g, 15.25 mmol) (pre-dissolved in anhydrous THF) was slowly added dropwise to carry out a reaction at room temperature for 3 h, 2 ml of a saturated ammonium chloride solution was added dropwise at - 78°C to quench the reaction (until a large amount of a solid was precipitated), then 3 ml of a 1 N NaOH solution was added and stirred at room temperature for 0.5 h, and suction filtration was performed to obtain a filtrate. Reduced pressure distillation was performed to remove the solvent, and a residue was purified by silica gel column chromatography (with an eluent: B system) to obtain (6-chloro-4-((3-morpholinopropyl)amino)pyridin-3-yl)methanol (3.1 g, a light yellow oily liquid) with a yield of 71.2 %. (3) The (6-chloro-4-((3-morpholinopropyl)amino)pyridin-3-yl)methanol (3 g, 10.50 mmol) was put into anhydrous dichloromethane, manganese dioxide (9.13 g, 104.98 mmol) was added to carry out a reaction overnight at room temperature, suction filtration was performed to remove the manganese dioxide and obtain a filtrate, reduced pressure distillation was performed to remove the solution, and a residue was purified by silica gel column chromatography (with an eluent: B system) to obtain 6-chloro-4-(3-(morpholinopropyl)amino)nicotinaldehyde (2.1 g, a light yellow oily liquid) with a yield of 71.4%. (4) The 6-chloro-4-(3-(morpholinopropyl)amino)nicotinaldehyde (1.5 g, 5.29 mmol), K₂CO₃ (2.19 g, 15.86 mmol) and ethyl 2-(3,5-dimethoxyphenyl)acetate (1.78 g, 7.93 mmol) were put into 10 ml of anhydrous N,N-dimethylformamide (DMF) to carry out a reaction under the protection of nitrogen at 100°C for 12 h. The reaction system was dissolved in a large amount of ethyl acetate and then washed with a saturated sodium chloride solution, reduced pressure distillation was performed to remove the solution, and a residue was purified by silica gel column chromatography (with an eluent: B system) to obtain 7-chloro-3-(3,5-dimethoxyphenyl)-1-(3-morpholinopropyl)-1,6-naphthyridin-2(1H)-one (1.5 g, a light yellow oily compound) with a yield of 65.9%. (5) The 7-chloro-3-(3,5-dimethoxyphenyl)-1-(3-morpholinopropyl)-1,6-naphthyridin-2(1H)-one (1 g, 2.25 mmol), 2-nitro-6-methylaniline (514.11 mg, 3.38 mmol), Pd₂(dba)₃ (206.28 mg, 225.26 µmol), BrettPhos (209.35 mg, 390.01 µmol) and cesium carbonate (1.91 g, 5.85 mmol) were put into 30 ml of toluene to carry out reflux under the protection of nitrogen at 120°C for 6 h. Reduced pressure distillation was performed to remove the solvent toluene, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(3,5-dimethoxyphenyl)-7-((2-methyl-6-nitrophenyl)amino)-1-(3-morpholinopropyl)-1,6-naphthyridin-2(1H)-one (766 mg, a reddish brown solid) with a yield of 62.3%. (6) The 3-(3,5-dimethoxyphenyl)-7-((2-methyl-6-nitrophenyl)amino)-1-(3-morpholinopropyl)-1,6-naphthyridin-2(1H)-one (500 mg, 893.46 µmol), di-tert-butyl dicarbonate (389.99 mg, 1.79 mmol) and 4-dimethylaminopyridine (10.92 mg, 89.35 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(3,5-dimethoxyphenyl)-1-(3-morpholinopropyl)-2-oxo-1,2-2H-1,6-naphthyridin-7-yl)(2-methyl-6-nitrophenyl)carbamate (403 mg, a light yellow solid) with a yield of 68.4%. (7) The tert-butyl (3-(3,5-dimethoxyphenyl)-1-(3-morpholinopropyl)-2-oxo-1,2-2H-1,6-naphthyridin-7-yl)(2-methyl-6-nitrophenyl)carbamate (350 mg, 530.51 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-nitrophenyl)(3-(3,5-dimethoxyphenyl)-2-oxo-1-(3-morpholinopropyl)-1,2-2H-1,6-naphthyridin-7-yl)carbamate (143 mg, a white solid) with a yield of 42.9%. (8) The tert-butyl (2-amino-6-nitrophenyl)(3-(3,5-dimethoxyphenyl)-2-oxo-1-(3-morpholinopropyl)-1,2-2H-1,6-naphthyridin-7-yl)carbamate (100 mg, 158.79 µmol) and triethylamine (88.05 mmL, 635.16 µmol) were dissolved in anhydrous THF, and then acryloyl chloride (25.81 mmL, 317.58 µmol) were added dropwise to carry out a reaction at room temperature for 2 h. A saturated ammonium chloride solution was added to quench the reaction, reduced pressure distillation was performed to remove the solvent, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (2-acrylamide-6-nitrophenyl)(3-(3,5-dimethoxyphenyl)-2-oxo-1-(3-morpholinopropyl)-2-oxo-1,2-2H-1,6-naphthyridin-7-yl)carbamate (74 mg, a white solid) with a yield of 68.6%. (9) The tert-butyl (2-acrylamide-6-nitrophenyl)(3-(3,5-dimethoxyphenyl)-2-oxo-1-(3-morpholinopropyl)-2-oxo-1,2-2H-1,6-naphthyridin-7-yl)carbamate (50 mg, 73.12 µmol) was dissolved in 4 ml of dichloromethane, and 1 ml of trifluoroacetic acid was added to carry out a reaction at room temperature for 2 h. A saturated sodium bicarbonate solution was added to quench the reaction, then an ethyl acetate/saturated sodium chloride solution was used for extraction for several times, and organic layers were combined. Reduced pressure distillation was performed to remove the solvent, and a residue was purified by silica gel column chromatography (with an eluent: B system) to obtain N-(2-((3-(3,5-dimethoxyphenyl)-1-(3-morpholinopropyl)-2-oxo-1,2-dihydro-1,6-naphthyridin-7-yl)amino)-3-methylphenyl)acrylamide (17 mg, a light yellow solid), recorded as a compound 26, with a yield of 40.5%. Characterization data are as follows: ¹H NMR (400 MHz, DMSO) δ 9.36 (s, 1H), 8.50 (s, 1H), 8.07 (s, 1H), 7.92 (s, 1H), 7.66 (d, J = 3.7 Hz, 1H), 7.19 (t, J = 7.8 Hz, 1H), 7.04 (d, J = 7.4 Hz, 1H), 6.83 (d, J = 2.2 Hz, 2H), 6.54 - 6.45 (m, 2H), 6.06 (d, J = 15.1 Hz, 1H), 5.57 (d, J = 12.1 Hz, 1H), 4.21 (t, J = 7.6 Hz, 2H), 3.73 (s, 6H), 2.48 - 2.44 (m, 6H), 2.40 (t, J = 6.4 Hz, 2H), 2.07 (s, 3H), 1.82 (d, J = 11.0 Hz, 2H).

**Example 6** A route and a preparation process are as follows.

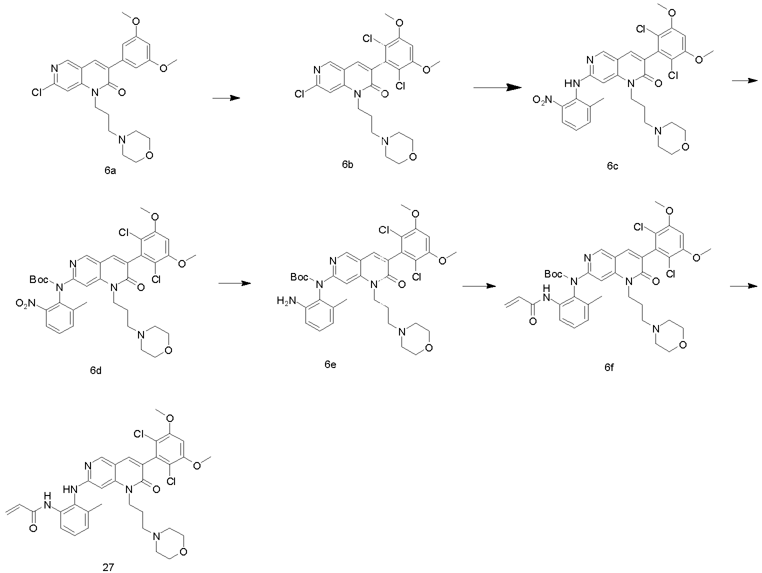

(1) 7-chloro-3-(3,5-dimethoxyphenyl)-1-(3-morpholinopropyl)-1,6-naphthyridin-2(1H)-one (1 g, 2.25 mmol) was dissolved in 50 ml of anhydrous acetonitrile and cooled to -20°C, and sulfonyl chloride (364.08 mmL, 4.51 mmol) (pre-dissolved in anhydrous acetonitrile) was added dropwise to carry out a reaction at room temperature for 10 min. A reaction solution was concentrated, 150 ml of dichloromethane was added, the pH value was adjusted to 8-9 with a saturated sodium bicarbonate solution, and layering was performed. An organic phase was concentrated, extraction was performed repeatedly with an ethyl acetate/distilled water system, and organic layers were combined. Reduced pressure distillation was performed to remove the solvent, and a residue was purified by silica gel column chromatography (with an eluent: B system) to obtain 7-chloro-3-(2,6-dichloro-3,5-dimethoxyphenyl)-1-(3-morpholinopropyl)-1,6-naphthyridin-2(1H)-one (884 mg, a white solid) with a yield of 76.2%. (2) The 7-chloro-3-(2,6-dichloro-3,5-dimethoxyphenyl)-1-(3-morpholinopropyl)-1,6-naphthyridin-2(1H)-one (800.00 mg, 1.56 mmol), 2-nitro-6-methylaniline (356.04 mg, 2.34 mmol), Pd₂(dba)₃ (142.86 mg, 156.00 µmol), BrettPhos (167.48 mg, 312.01 µmol) and cesium carbonate (1.52 g, 4.68 mmol) were put into 30 ml of toluene to carry out reflux under the protection of nitrogen at 120°C for 6 h. Reduced pressure distillation was performed to remove the solvent, extraction was performed repeatedly with an ethyl acetate/saturated sodium chloride system, and organic layers were combined. Reduced pressure distillation was performed to remove the solvent, and a residue was purified by silica gel column chromatography (with an eluent: B system) to obtain 3-(2,6-dichloro-3,5-dimethoxyphenyl)-7-((2-methyl-6-nitrophenyl)amino)-1-(3-morpholinopropyl)-1,6-naphthyridin-2(1H)-one (552 mg, a reddish brown solid) with a yield of 56.3%. (3) The 3-(2,6-dichloro-3,5-dimethoxyphenyl)-7-((2-methyl-6-nitrophenyl)amino)-1-(3-morpholinopropyl)-1,6-naphthyridin-2(1H)-one (500 mg, 795.54 µmol), di-tert-butyl dicarbonate (347.25 mg, 1.59 mmol) and 4-dimethylaminopyridine (9.72 mg, 79.55 µmol) were put into anhydrous dichloromethane to carry out a reaction at room temperature for 4 h, the reaction system was extracted for several times with an ethyl acetate/saturated sodium chloride solution, and organic layers were combined. Reduced pressure distillation was performed to remove the solvent, and a residue was purified by silica gel column chromatography (with an eluent: B system) to obtain tert-butyl (3-(2,6-dichloro-3,5-dimethoxyphenyl)-1-(3-morpholinopropyl)-2-oxo-1,2-2H-1,6-naphthyridin-7-yl)(2-methyl-6-nitrophenyl)carbamate (372 mg, a light yellow solid) with a yield of 64.3%. (4) The tert-butyl (3-(2,6-dichloro-3,5-dimethoxyphenyl)-1-(3-morpholinopropyl)-2-oxo-1,2-2H-1,6-naphthyridin-7-yl)(2-methyl-6-nitrophenyl)carbamate (300 mg, 411.73 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-methylphenyl)(3-(2,6-dichloro-3,5-dimethoxyphenyl)-1-(3-morpholinopropyl)-2-oxo-1,2-2H-1,6-naphthyridin-7-yl)carbamate (165 mg, a white solid) with a yield of 57.6%. (5) The tert-butyl (2-amino-6-methylphenyl)(3-(2,6-dichloro-3,5-dimethoxyphenyl)-1-(3-morpholinopropyl)-2-oxo-1,2-2H-1,6-naphthyridin-7-yl)carbamate (100 mg, 143.13 µmol) and triethylamine (79.37 mmL, 572.54 µmol) were dissolved in anhydrous THF, and then acryloyl chloride (23.27 mmL, 286.27 µmol) was added dropwise to carry out a reaction at room temperature for 2 h. A saturated ammonium chloride solution was added to quench the reaction, reduced pressure distillation was performed to remove the solvent, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (2-acrylamide-6-methylphenyl)(3-(2,6-dichloro-3,5-dimethoxyphenyl)-1-(3-morpholinopropyl)-2-oxo-1,2-2H-1,6-naphthyridin-7-yl)carbamate (84 mg, a white solid) with a yield of 78.2%. (6) The tert-butyl (2-acrylamide-6-methylphenyl)(3-(2,6-dichloro-3,5-dimethoxyphenyl)-1-(3-morpholinopropyl)-2-oxo-1,2-2H-1,6-naphthyridin-7-yl)carbamate (50.00 mg, 66.43 µmol) was dissolved in 4 ml of dichloromethane, and 1 ml of trifluoroacetic acid was added to carry out a reaction at room temperature for 2 h. A saturated sodium bicarbonate solution was added to quench the reaction, and then an ethyl acetate/saturated sodium chloride solution was used for extraction for several times. Reduced pressure distillation was performed to remove the solvent, and a residue was purified by silica gel column chromatography (with an eluent: B system) to obtain N-(2-((3-(2,6-dichloro-3,5-dimethoxyphenyl)-1-(3-morpholinopropyl)-2-oxo-1,2-2H-1,6-naphthyridin-7-yl)amino)-3-methylphenyl)acrylamide (23 mg, a light yellow solid), recorded as a compound 27, with a yield of 54.2%. Characterization data are as follows: ¹H NMR (400 MHz, DMSO) δ 9.40 (s, 1H), 8.55 (s, 1H), 8.12 (s, 1H), 7.96 (s, 1H), 7.71 (d, J = 7.4 Hz, 1H), 7.23 (t, J = 7.8 Hz, 1H), 7.08 (d, J = 7.4 Hz, 1H), 6.88 (d, J = 2.2 Hz, 2H), 6.57 - 6.49 (m, 2H), 6.10 (dd, J = 16.0, 1.9 Hz, 1H), 5.61 (dd, J = 12.0, 4.0 Hz, 1H), 4.25 (t, J = 7.6 Hz, 2H), 3.77 (s, 6H), 3.49 (s, 1H), 3.41 (dd, J = 7.8, 2.9 Hz, 2H), 2.44 (t, J = 6.4 Hz, 2H), 2.23 (s, 6H), 2.11 (s, 3H).

**Example 7** A route and a preparation process are as follows.

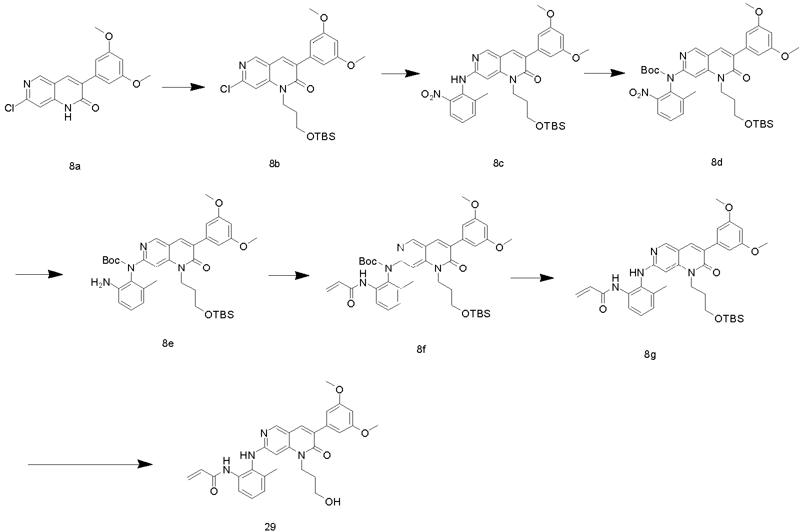

(1) 7-chloro-3-(3,5-dimethoxyphenyl)-1,6-naphthyridin-2(1H)-aldehyde (900 mg, 2.84 mmol) was dissolved in 10 ml of dimethyl sulfoxide (DMSO), K₂CO₃ (706.86 mg, 5.11 mmol) was added at room temperature and stirred for 10 min, 1-(tert-butyl-dimethylsilyl)-3-chloropropane (949.27 mg, 4.55 mmol) was added and heated to 90°C to carry out a reaction for 5 h, 200 ml of ethyl acetate was added, and washing was performed repeatedly for 2 times with 20 ml of ultrapure water. Reduced pressure distillation was performed to remove the solvent, and a residue was purified by silica gel column chromatography (with an eluent: B system) to obtain 1-(3-((tert-butyl-dimethylsilyl)oxy)propyl)-7-chloro-3-(3,5-dimethoxyphenyl)-1,6-naphthyridin-2(1H)-aldehyde (863 mg, a white solid) with a yield of 62.1%. (2) The 1-(3-((tert-butyl-dimethylsilyl)oxy)propyl)-7-chloro-3-(3,5-dimethoxyphenyl)-1,6-naphthyridin-2(1H)-aldehyde (800 mg, 1.64 mmol), 2-nitro-6-methylaniline (373.32 mg, 2.45 mmol), Pd₂(dba)₃ (149.79 mg, 163.57 µmol), BrettPhos (175.60 mg, 327.14 µmol) and cesium carbonate (1.60 g, 4.91 mmol) were put into 25 ml of toluene to carry out reflux under the protection of nitrogen at 120°C for 7 h. Reduced pressure distillation was performed to remove the solvent, and extraction was performed for several times with an ethyl acetate/saturated sodium chloride system. Reduced pressure distillation was performed to remove the solvent, and a residue was purified by silica gel column chromatography (with an eluent: B system) to obtain 1-(3-((tert-butyldimethylsilyl)oxy)propyl)-3-(2,6-dichloro-3,5-dimethoxyphenyl)-7-((2-methyl-6-nitrophenyl)amino)-1,6-naphthyridin-2(1H)-aldehyde (477 mg, a brownish yellow solid) with a yield of 48.2%. (3) The 1-(3-((tert-butyl-dimethylsilyl)oxy)propyl)-3-(2,6-dichloro-3,5-dimethoxyphenyl)-7-((2-methyl-6-nitrophenyl)amino)-1,6-naphthyridin-2(1H)-aldehyde (450 mg, 744.07 µmol), di-tert-butyl dicarbonate (181.81 mg, 1.49 mmol) and 4-dimethylaminopyridine (32.48 mg, 148.81 µmol) were put into anhydrous dichloromethane to carry out a reaction at room temperature for 4 h, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (1-(3-((tert-butyldimethylsilyl)oxy)propyl)-3-(3,5-dimethoxyphenyl)-2-oxo-1,2-2H-1,6-naphthyridin-7-yl)(2-methyl-6-nitrophenyl)carbamate (338 mg, a light yellow solid) with a yield of 64.5%. (4) The tert-butyl (1-(3-((tert-butyl-dimethylsilyl)oxy)propyl)-3-(3,5-dimethoxyphenyl)-2-oxo-1,2-2H-1,6-naphthyridin-7-yl)(2-methyl-6-nitrophenyl)carbamate (300 mg, 425.59 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-methylphenyl)(1-(3-((tert-butyl-dimethylsilyl)oxy)propyl)-3-(3,5-dimethoxyphenyl)-2-oxo-1,2-2H-1,6-naphthyridin-7-yl)carbamate (138 mg, a white solid) with a yield of 48.2%. (5) The tert-butyl (2-amino-6-methylphenyl)(1-(3-((tert-butyl-dimethylsilyl)oxy)propyl)-3-(3,5-dimethoxyphenyl)-2-oxo-1,2-2H-1,6-naphthyridin-7-yl)carbamate (100 mg, 148.17 µmol) and triethylamine (82.16 mmL, 592.67 µmol) were dissolved in anhydrous THF, and then acryloyl chloride (24.08 mmL, 296.33 µmol) was added dropwise to carry out a reaction at room temperature for 2 h. A saturated ammonium chloride solution was added to quench the reaction, reduced pressure distillation was performed to remove the solvent, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (2-acrylamide-6-methylphenyl)(1-(3-((tert-butyl-dimethylsilyl)oxy)propyl)-3-(3,5-dimethoxyphenyl)-2-oxo-1,2-2H-1,6-naphthyridin-7-yl)carbamate (56 mg, a white solid) with a yield of 52.3%. (6) The tert-butyl (2-acrylamide-6-methylphenyl)(1-(3-((tert-butyl-dimethylsilyl)oxy)propyl)-3-(3,5-dimethoxyphenyl)-2-oxo-1,2-2H-1,6-naphthyridin-7-yl)carbamate (50 mg, 68.59 µmol) was dissolved in 10 ml of dichloromethane, and 1 ml of trifluoroacetic acid was added to carry out a reaction at room temperature for 2 h. A saturated sodium bicarbonate solution was added to quench the reaction, and then an ethyl acetate/saturated sodium chloride solution was used for extraction for several times. Reduced pressure distillation was performed to remove the solvent, and a residue was purified by silica gel column chromatography (with an eluent: B system) to obtain N-(2-((3-(3,5-dimethoxyphenyl)-1-(3-hydroxyethyl)-2-oxo-1,2-2H-1,6-naphthyridin-7-yl)amino)-3-methylphenyl)acrylamide (15 mg, a light yellow solid), recorded as a compound 29, with a yield of 42.6%. Characterization data are as follows: ¹H NMR (400 MHz, DMSO) δ 9.49 (s, 1H), 8.41 (s, 1H), 8.28 (s,1H), 7.97 (s, 1H), 7.68 (d, J = 7.7 Hz, 1H), 7.17(t, J = 7.8 Hz, 1H), 7.08 (d, J = 3.0 Hz, 1H), 6.80 (s, 2H), 6.45 (s, 1H), 6.15 (d, J =18.9 Hz, 2H), 5.63 (d, J = 12.1 Hz, 1H), 4.07-3.96 (m, 2H), 3.73 (s, 6H), 3.40 (s, 2H), 2.13 (s, 4H), 1.69-1.59 (m, 2H).

**Example 8** A route and a preparation process are as follows.

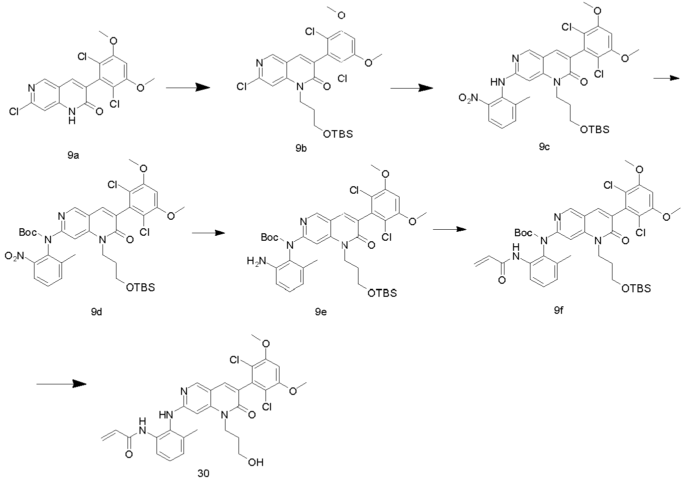

(1) 7-chloro-3-(3,5-dimethoxyphenyl)-1,6-naphthyridin-2(1H)-aldehyde (900 mg, 2.33 mmol) was dissolved in 10 ml of dimethyl sulfoxide (DMSO), K₂CO₃ (580.59 mg, 4.20 mmol) was added at room temperature and stirred for 10 min, 1-(tert-butyl-dimethylsilyl)-3-chloropropane (876.07 mmL, 3.73 mmol) was added and heated to 90°C to carry out a reaction for 5 h, 200 ml of ethyl acetate was added, and washing was performed repeatedly for 2 times with 20 ml of ultrapure water. Reduced pressure distillation was performed to remove the solvent, and a residue was purified by silica gel column chromatography (with an eluent: B system) to obtain 1-(3-((tert-butyl-dimethylsilyl)oxy)propyl)-7-chloro-3-(2,6-dichloro-3,5-dimethoxyphenyl)-1,6-naphthyridin-2(1H)-aldehyde (890 mg, a white solid) with a yield of 68.4%. (2) The 1-(3-((tert-butyl-dimethylsilyl)oxy)propyl)-7-chloro-3-(2,6-dichloro-3,5-dimethoxyphenyl)-1,6-naphthyridin-2(1H)-aldehyde (800 mg, 1.43 mmol), 2-nitro-6-methylaniline (327.23 mg, 2.15 mmol), Pd₂(dba)₃ (131.30 mg, 143.38 µmol), BrettPhos (153.92 mg, 286.75 µmol) and cesium carbonate (1.40 g, 4.30 mmol) were put into 25 ml of toluene to carry out reflux under the protection of nitrogen at 120°C for 7 h. Reduced pressure distillation was performed to remove the solvent, and extraction was performed for several times with an ethyl acetate/saturated sodium chloride system. Reduced pressure distillation was performed to remove the solvent, and a residue was purified by silica gel column chromatography (with an eluent: B system) to obtain 1-(3-((tert-butyldimethylsilyl)oxy)propyl)-3-(2,6-dichloro-3,5-dimethoxyphenyl)-7-chloro-((2-methyl-6-nitrophenyl)amino)-1,6-naphthyridin-2(1H)-aldehyde (620 mg, a brownish yellow solid) with a yield of 64.24%. (3) The 1-(3-((tert-butyl-dimethylsilyl)oxy)propyl)-3-(2,6-dichloro-3,5-dimethoxyphenyl)-7-chloro-((2-methyl-6-nitrophenyl)amino)-1,6-naphthyridin-2(1H)-aldehyde (600 mg, 890.65 µmol), di-tert-butyl dicarbonate (217.6 mg, 1.78 mmol) and 4-dimethylaminopyridine (38.88 mg, 178.13 µmol) were put into anhydrous dichloromethane to carry out a reaction at room temperature for 4 h, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (1-(3-((tert-butyldimethylsilyl)oxy)propyl)-3-(2,6-dichloro-3,5-dimethoxyphenyl)-2-oxo-1,2-2H-1,6-naphthyridin-7-yl)(2-methyl-6-nitrophenyl)carbamate (480 mg, a light yellow solid) with a yield of 69.6%. (4) The tert-butyl (1-(3-((tert-butyl-dimethylsilyl)oxy)propyl)-3-(2,6-dichloro-3,5-dimethoxyphenyl)-2-oxo-1,2-2H-1,6-naphthyridin-7-yl)(2-methyl-6-nitrophenyl)carbamate (300.00 mg, 268.89 µmol) was dissolved in an anhydrous THF solution, 20% of Pd/C was added to carry out a reduction reaction at room temperature for 48 h under hydrogen conditions, suction filtration was performed to remove the palladium on carbon, a filtrate was subjected to reduced pressure distillation to remove the solvent, and a residue was purified by silica gel column chromatography (with an eluent: B system) to obtain tert-butyl (2-amino-6-methylphenyl)(1-(3-((tert-butyl-dimethylsilyl)oxy)propyl)-3-(2,6-dichloro-3,5-dimethoxyphenyl)-2-oxo-1,2-2H-1,6-naphthyridin-7-yl)carbamate (150 mg, a white solid) with a yield of 52.01%. (5) The tert-butyl (2-amino-6-methylphenyl)(1-(3-((tert-butyldimethylsilyl)oxy)propyl)-3-(2,6-dichloro-3,5-dimethoxyphenyl)-2-oxo-1,2-2H-1,6-naphthyridin-7-yl)carbamate (100 mg, 134.45 µmol) and triethylamine (74.55 mmL, 537.78 µmol) were dissolved in anhydrous THF, and then acryloyl chloride (21.85 mmL, 268.89 µmol) was added dropwise to carry out a reaction at room temperature for 2 h. A saturated ammonium chloride solution was added to quench the reaction, reduced pressure distillation was performed to remove the solvent, and the reaction system was extracted for several times with an ethyl acetate/saturated sodium chloride solution. Reduced pressure distillation was performed to remove the solvent, and a residue was purified by silica gel column chromatography (with an eluent: B system) to obtain tert-butyl (2-acrylamide-6-methylphenyl)(1-(3-((tert-butyldimethylsilyl)oxy)propyl)-3-(2,6-dichloro-3,5-dimethoxyphenyl)-2-oxo-1,2-2H-1,6-naphthyridin-7-yl)carbamate (80 mg, a white solid) with a yield of 83.90%. (6) The tert-butyl (2-acrylamide-6-methylphenyl)(1-(3-((tert-butyl-dimethylsilyl)oxy)propyl)-3-(2,6-dichloro-3,5-dimethoxyphenyl)-2-oxo-1,2-2H-1,6-naphthyridin-7-yl)carbamate (60 mg, 75.20 µmol) was dissolved in 10ml of dichloromethane, and 1 ml of trifluoroacetic acid was added to carry out a reaction at room temperature for 2 h. A saturated sodium bicarbonate solution was added to quench the reaction, and then an ethyl acetate/saturated sodium chloride solution was used for extraction for several times. Reduced pressure distillation was performed to remove the solvent, and a residue was purified by silica gel column chromatography (with an eluent: B system) to obtain N-(2-((3-(2,6-dichloro-3,5-dimethoxyphenyl)-1-(3-hydroxypropyl)-2-oxo-1,2-2H-1,6-naphthyridin-7-yl)amino)-3-methylphenyl)acrylamide (35 mg, a light yellow solid), recorded as a compound 30, with a yield of 79.76%. Characterization data are as follows: ¹H NMR (400 MHz, DMSO) δ 8.74 (s, 1H), 8.15 (s, 1H), 7.68 (s, 2H), 7.12 (d, J = 7.5 Hz, 1H), 6.76 (d, J = 2.2 Hz, 2H), 6.48 (s, 1H), 6.38 (d, J = 15.8 Hz, 1H), 6.29 (d, J = 9.8 Hz, 1H), 6.15 (s, 1H), 5.71 (d, J = 10.1 Hz, 1H), 4.05 (s, 2H), 3.80 (s, 6H), 2.23 (s, 3H), 1.62 (m, 2H).

### Example 9 Biological activity screening

The inhibition activity of compounds 22-30 on FGFR1-4 kinase was tested by a Mobility shift assay.

A specific experimental method is as follows.

(1) A 1×Kinase buffer was prepared. (2) Preparation of the compounds based on concentration: A test concentration of each compound was 300 nM, the compound was diluted for 3 times, 8 or 9 concentrations were used, and single hole detection was adopted. The compound was diluted to a 100 times final concentration with a 100% DMSO solution in a 384 source plate. 250 nL of the compound with a 100 times final concentration was transferred to a target plate, 3573 plate, by using a liquid separator Echo 550. (3) A kinase solution with a 2.5 times final concentration was prepared with the 1×Kinase buffer. (4) 10 µL of the kinase solution with a 2.5 times final concentration was added to compound holes and positive control holes, respectively; and 10 µL of the 1×Kinase buffer was added to negative control holes. (5) Centrifugation was performed at 1,000 rpm for 30 s, the reaction plate was shaken for even mixing, and incubation was performed at room temperature for 60 min. (6) A mixed solution, with a 25/15 times final concentration, of ATP and Kinase substrate22 was prepared with the 1×Kinase buffer. (7) 15 µL of a mixed solution, with a 5/3 times final concentration, of ATP and a substrate was added to carry out an initial reaction. (8) A 384-well plate was centrifuged at 1,000 rpm for 30 s and shaken for even mixing, and incubation was performed at room temperature for 30 min. (9) 30 µL of a terminal detection solution was added to stop a kinase reaction, centrifugation was performed at 1,000 rpm for 30 s, and shaking was performed for even mixing. (10) A conversion rate was read with Caliper EZ Reader.

Data analysis is as follows.

A calculation formula is: Inhibition (%)=(Conversion%_max-Conversion%_sample)/(Conversion%_max-Conversion%_min)x100,
where Conversion%_sample is a conversion rate read value of a sample; Conversion%_min is an average value of the negative control holes, representing a conversion rate read value of holes without enzyme activity; and Conversion%_max is an average value of the positive control holes, representing a conversion rate read value of holes without

### compound inhibition.

A dose-effect curve is fitted as follows.

With a concentration log value as an X axis and an inhibition rate percentage as a Y axis, the dose-effect curve was fitted by log (inhibitor) vs. response-Variable slope of analysis software GraphPad Prism 5, so as to obtain an IC50 value of each compound on enzyme activity. A calculation formula is: Y=Bottom+(Top-Bottom)/(1+10^((LogIC50-X)*HillSlope)).

### Experimental results

| Compound | **IC50 (nM)** | | | |
|---|---|---|---|---|
| | **FGFR4** | **FGFR1** | **FGFR2** | **FGFR3** |
| **22** | **9.8** | **>5000** | **>5000** | **>5000** |
| **23** | **3.7** | **>5000** | **>5000** | **>5000** |
| **24** | **34.4** | **>5000** | **>5000** | **>5000** |
| **25** | **5.8** | **>5000** | **>5000** | **>5000** |
| **26** | **20.1** | **>5000** | **>5000** | **>5000** |
| **27** | **4.4** | **>5000** | **>5000** | **>5000** |
| **29** | **31.5** | **>5000** | **>5000** | **>5000** |
| **30** | **5.9** | **>5000** | **>5000** | **>5000** |
| **BLU9931** | **4.5** | **582.5** | **436.4** | **193.2** |
| **BGJ398** | **57.3** | **0.72** | **1.8** | **0.57** |

The experimental results show that the series of compounds 22-30 can effectively inhibit the expression of FGFR4, basically have no inhibition activity on homologous kinase FGFR-3, and have better kinase selectivity compared with FGFR inhibitors, BLU9931 and BGJ398. The BLU9931 and the BGJ398 have structural formulas as follows:

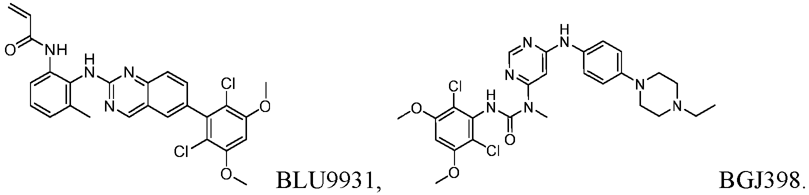

### Example 10 Effect of active compounds on proliferation of hepatoma carcinoma cells HepG2, HuH-7 and MiHA

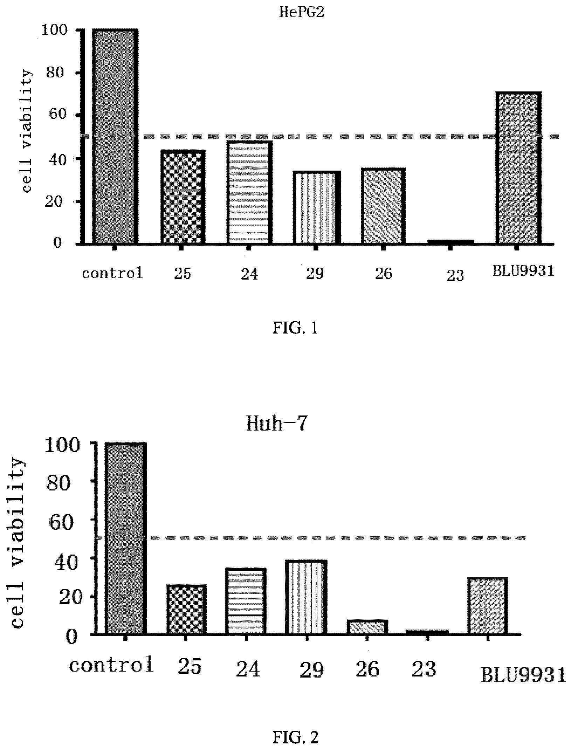

An MTT method was used for determination. An experimental method is briefly described as follows. A test compound was first dissolved in DMSO to prepare a stock solution, and then the stock solution was subjected to gradient dilution with a corresponding cell culture medium to prepare a test sample, where a final concentration of the compound was 20 uM. Cells in a logarithmic culture stage were inoculated into a 96-well cell culture plate at an appropriate density, cultured overnight under corresponding conditions, and then continuously cultured for 72 h after the test compound sample was added. After the culture was completed, an appropriate volume of an MTT detection solution was added to each well, incubation was performed at 37°C for 1-4 h, and the culture medium was carefully discarded. Then, 100 µL of DMSO was added to each well to dissolve a crystal at the bottom of the hole, and shaking was performed on a microoscillator under dark conditions at room temperature for 10 min. After the crystals were fully dissolved, a light absorption value of each well at 560 nm was detected by a microplate reader, and an inhibition rate of each compound on hepatoma carcinoma cells was calculated. Results are shown in FIG. 1 to FIG. 3. As can be seen from FIG. 1 to FIG. 3, the preferred compounds of the present invention have an obvious proliferation inhibition effect on hepatoma carcinoma cells with FGFR4 abnormality.

### Example 11 Effect of an active compound on proliferation of colon cancer cells HCT-116, SW480 and RKO

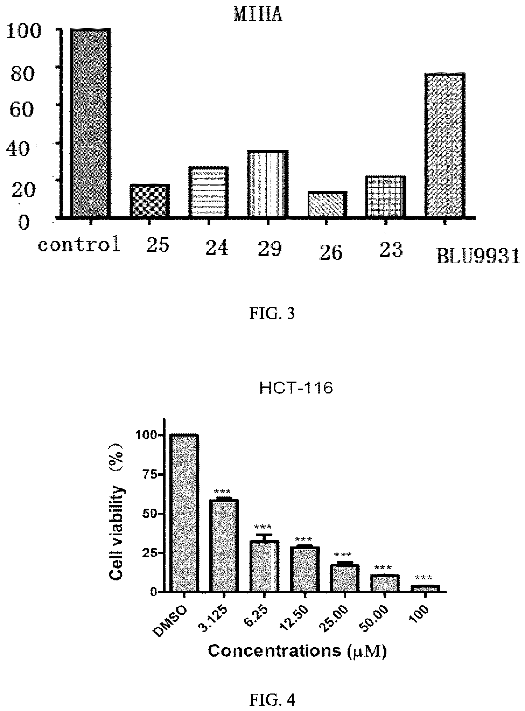

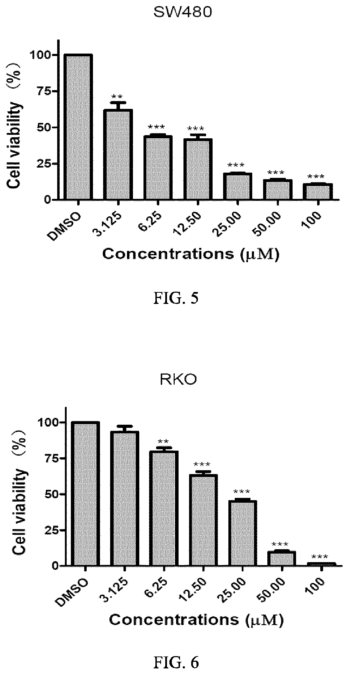

An MTT method was used for determination. An experimental method is briefly described as follows. A test compound 23 was first dissolved in DMSO to prepare a stock solution, and then the stock solution was subjected to gradient dilution with a corresponding cell culture medium to prepare a test sample, where a final concentration of the compound was in the range of 3-100 uM. Cells in a logarithmic culture stage were inoculated into a 96-well cell culture plate at an appropriate density, cultured overnight under corresponding conditions, and then continuously cultured for 72 h after the test compound sample was added. After the culture was completed, an appropriate volume of an MTT detection solution was added to each well, incubation was performed at 37°C for 1-4 h, and the culture medium was carefully discarded. Then, 100 µL of DMSO was added to each well to dissolve a crystal at the bottom of the hole, and shaking was performed on a microoscillator under dark conditions at room temperature for 10 min. After the crystals were fully dissolved, a light absorption value of each well at 560 nm was detected by a microplate reader, and an inhibition rate of the compound on colon cancer cells was calculated. Results are shown in FIG. 4 to FIG. 6. As can be seen from FIG. 4 to FIG. 6, the compound 23 of the present invention can inhibit proliferation of 3 kinds of colon cancer cells HCT-116, SW480 and RKO at a concentration of 3-100 µM in a dose-dependent method, and has good activity against colon cancer.

## Claims

1. A 2-oxo-1,2-dihydro-1,6-naphthyridin-7-yl compound, **characterized in that** the compound is one of the following compounds, stereoisomers thereof, tautomers thereof, or pharmaceutically acceptable salts thereof:

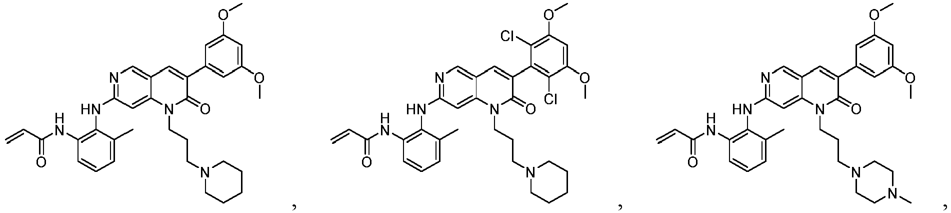

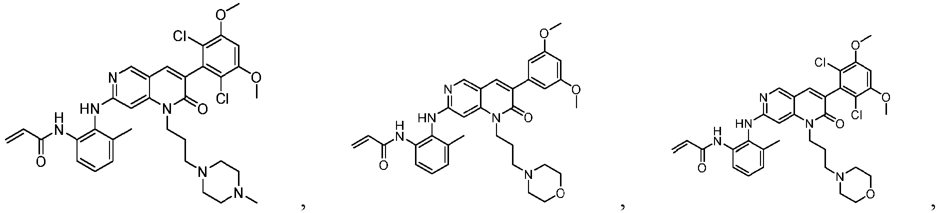

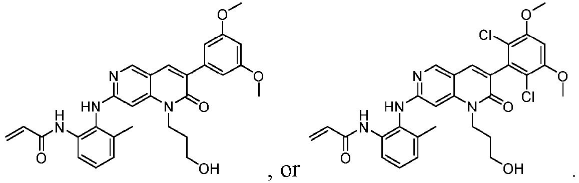

2. The 2-oxo-1,2-dihydro-1,6-naphthyridin-7-yl compound according to claim 1 for use as drugs, **characterized in that** the drugs are antitumor drugs.

3. The 2-oxo-1,2-dihydro-1,6-naphthyridin-7-yl compound for use according to claim 2, **characterized in that** the antitumor drugs use FGFR4 as a target.

4. The 2-oxo-1,2-dihydro-1,6-naphthyridin-7-yl compound for use according to claim 3, **characterized in that** the antitumor drugs selectively inhibit FGFR4, and have almost no inhibition on FGFR1, FGFR2 and FGFR3.

5. The 2-oxo-1,2-dihydro-1,6-naphthyridin-7-yl compound for use according to claim 2, **characterized in that** the drugs are used for treating liver cancer, carcinoma of bile duct, breast cancer, or urothelium carcinoma.

6. The 2-oxo-1,2-dihydro-1,6-naphthyridin-7-yl compound for use according to claim 2, **characterized in that** the drugs are used for treating one or more of leukaemia, colon cancer, rectal cancer, gastric cancer, ovarian cancer, chorionic epithelioma, malignant mole, head and neck squamous cell carcinoma, skin cancer, bladder cancer, lung cancer, prostate cancer, uterine cancer, kidney cancer, lymphoma, or cervical cancer.

## Patentansprüche

1. Eine 2-Oxo-1,2-dihydro-1,6-naphthyridin-7-yl-Verbindung, **dadurch gekennzeichnet, dass** die Verbindung eine der folgenden Verbindungen, deren Stereoisomere, deren Tautomere oder deren pharmazeutisch verträgliche Salze ist:

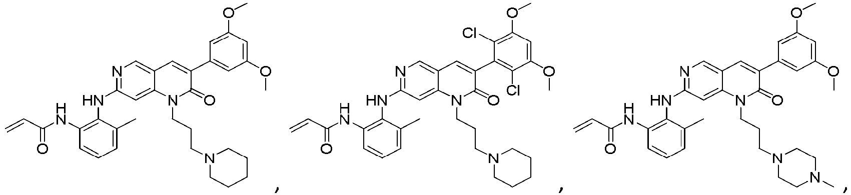

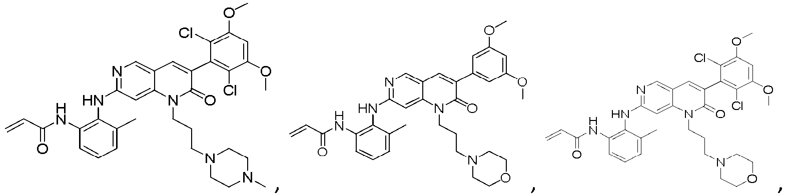

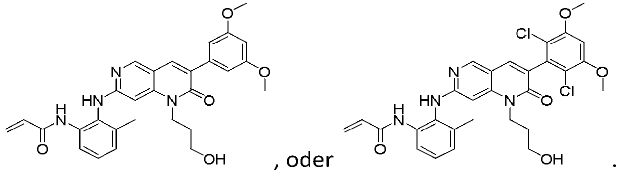

2. 2-Oxo-1,2-dihydro-1,6-naphthyridin-7-yl-Verbindung gemäß Anspruch 1 zur Verwendung als Arzneimittel, **dadurch gekennzeichnet, dass** die Arzneimittel Antitumormittel sind.

3. 2-Oxo-1,2-dihydro-1,6-naphthyridin-7-yl-Verbindung zur Verwendung gemäß Anspruch 2, **dadurch gekennzeichnet, dass** die Antitumormittel FGFR4 als Ziel verwenden.

4. 2-Oxo-1,2-dihydro-1,6-naphthyridin-7-yl-Verbindung zur Verwendung gemäß Anspruch 3, **dadurch gekennzeichnet, dass** die Antitumormittel FGFR4 selektiv hemmen und FGFR1, FGFR2 und FGFR3 fast nicht hemmen.

5. 2-Oxo-1,2-dihydro-1,6-naphthyridin-7-yl-Verbindung zur Verwendung gemäß Anspruch 2, **dadurch gekennzeichnet, dass** die Arzneimittel zur Behandlung von Leberkrebs, Gallengangskarzinom, Brustkrebs oder Urothelkarzinom verwendet werden.

6. 2-Oxo-1,2-dihydro-1,6-naphthyridin-7-yl-Verbindung zur Verwendung gemäß Anspruch 2, **dadurch gekennzeichnet, dass** die Arzneimittel zur Behandlung von einer oder mehreren der folgenden Erkrankungen verwendet werden: Leukämie, Darmkrebs, Rektumkrebs, Magenkrebs, Eierstockkrebs, Chorionepitheliom, maligner Muttermale, Plattenepithelkarzinomen im Kopf- und Halsbereich, Hautkrebs, Blasenkrebs, Lungenkrebs, Prostatakrebs, Gebärmutterkrebs, Nierenkrebs, Lymphomen oder Gebärmutterhalskrebs.

## Revendications

1. Composé 2-oxo-1,2-dihydro-1,6-naphtyridin-7-yle, **caractérisé en ce que** le composé est l'un des composés suivants, stéréo-isomères de celui-ci, tautomères de celui-ci ou sels pharmaceutiquement acceptables de celui-ci :

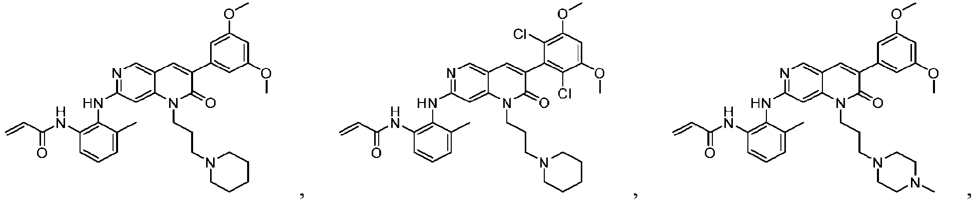

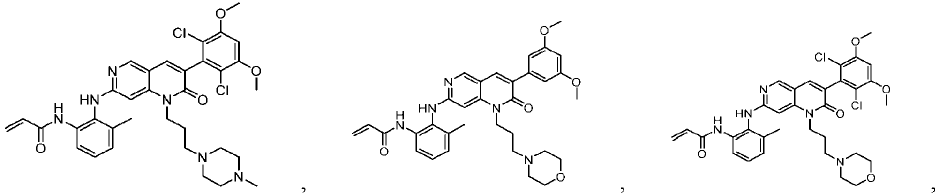

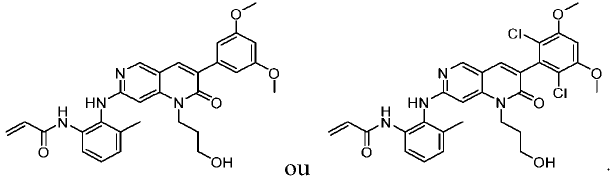

2. Composé 2-oxo-1,2-dihydro-1,6-naphtyridin-7-yle selon la revendication 1 pour utilisation comme médicaments, **caractérisé en ce que** les médicaments sont des médicaments antitumoraux.

3. Composé 2-oxo-1,2-dihydro-1,6-naphtyridin-7-yle pour utilisation selon la revendication 2, **caractérisé en ce que** les médicaments antitumoraux ont le FGFR4 pour cible.

4. Composé 2-oxo-1,2-dihydro-1,6-naphtyridin-7-yle pour utilisation selon la revendication 3, **caractérisé en ce que** les médicaments antitumoraux inhibent sélectivement le FGFR4 et n'ont pratiquement aucune inhibition sur le FGFR1, le FGFR2 et le FGFR3.

5. Composé 2-oxo-1,2-dihydro-1,6-naphtyridin-7-yle pour utilisation selon la revendication 2, **caractérisé en ce que** les médicaments sont utilisés pour traiter le cancer du foie, le carcinome des voies biliaires, le cancer du sein ou le carcinome urothélial.

6. Composé 2-oxo-1,2-dihydro-1,6-naphtyridin-7-yle pour utilisation selon la revendication 2, **caractérisé en ce que** les médicaments sont utilisés pour traiter une ou plusieurs affections parmi la leucémie, le cancer du côlon, le cancer du rectum, le cancer de l'estomac, le cancer de l'ovaire, le choriocarcinome, le mélanome malin, le carcinome épidermoïde de la tête et du cou, le cancer de la peau, le cancer de la vessie, le cancer du poumon, le cancer de la prostate, le cancer de l'utérus, le cancer du rein, le lymphome, ou le cancer du col de l'utérus.
